# EUROPÄISCHE PATENTSCHRIFT

(11) **EP 0 865 488 B1**
(45) Veröffentlichungstag und Bekanntmachung des Hinweises auf die Patenterteilung: **09.03.2005**
(21) Anmeldenummer: 96942330.0
(22) Anmeldetag: 04.12.1996
(51) Int. Cl.: C12N 15/10, C12N 9/24, C12Q 1/68, C12P 19/34

(54) **THERMOLABILE URACIL-DNA-GLYKOSYLASE, VERFAHREN ZU DEREN HERSTELLUNG UND VERWENDUNG ZUR ENTFERNUNG VON URACIL AUS DNA**
THERMOLABILE URACIL-DNA-GLYCOSYLASE, PROCESS FOR ITS PREPARATION AND USE FOR REMOVING URACIL FROM DNA
URACILE-ADN-GLYCOSYLASE THERMOLABILE, SON PROCEDE DE PREPARATION ET SON UTILISATION POUR ELIMINER L'URACILE DE L'ADN

(30) Priorität: 05.12.1995 DE 19545320
(43) Veröffentlichungstag der Anmeldung: 23.09.1998
(73) Patentinhaber: Roche Diagnostics GmbH, 68298 Mannheim (DE)
(72) Erfinder: SOBEK, Harald, D-82377 Penzberg (DE); SCHMIDT, Manfred, D-82377 Penzberg (DE); FREY, Bruno, D-82377 Penzberg (DE); KALUZA, Klaus, D-83670 Bad Heilbrunn (DE)
(86) Internationale Anmeldenummer: PCT/EP1996/005398
(87) Internationale Veröffentlichungsnummer: WO 1997/020922

(56) Entgegenhaltungen:
- WO-A-92/01814
- BIOCHIM BIOPHYS ACTA, NOV 21 1991, 1097 (4) P299-308, NETHERLANDS, XP000651732 SEAL G ET AL: "Purification and properties of the *uracil* *DNA* *glycosylase* from Bloom's syndrome."
- VIROLOGY, Bd. 198, 1994, Seiten 504-513, XP002029674 MILLNS, A.K. ET AL.: "The vaccinia virus-encoded uracil DNA glycosylase has an essential role in viral DNA replication"

## Beschreibung

Die Erfindung betrifft ein thermolabiles (hitzelabiles) Enzym mit Uracil-DNA-Glykosylase-Aktivität, ein Verfahren zur Gewinnung des Enzyms aus Gramm-positiven Mikroorganismen sowie ein verbessertes Verfahren zum Nachweis bzw. Entfernung von Uracil aus uracilhaltiger DNA, insbesondere aus DNA-Fragmenten, die nach spezifischer Amplifikation (z.B. PCR) erhalten werden.

Uracil-DNA-Glykosylasen (UNG; EC 3.2.2.3) sind weit verbreitete, hochkonservierte und extrem spezifische DNA-Reparaturenzyme. Ihre biologische Funktion ist die spezifische Entfernung der Base Uracil aus DNA. Uracil kann in DNA durch die spontane Deaminierung von Cytosin, oder durch die Mißincorporation von dUTP während der DNA-Synthese entstehen. Deaminierung von Cytosin führt zu promutagenen U:G Falschpaarungen, die, wenn sie nicht korrigiert werden, zu Transitions-Mutationen in der nächsten Runde der DNA-Synthese führen (Lindahl, T. (1993) Nature 362, 709-715).

UNGs werden insbesondere im Rahmen der PCR-Technologie bei der Dekontaminierung von PCR-Ansätzen verwendet. Die sogenannte "carry-over"-Kontaminierung von PCR-Ansätzen durch amplifizierte Target-DNA kann zu falsch-positiven Resultaten führen. Die "carry-oyer"-Kontaminierung kann durch den Einbau von dUTP in alle PCR-Produkte (wobei dTTP durch dUTP ersetzt wird) und die Behandlung von fertig gemixten PCR-Reaktionen mit UNG, gefolgt von thermischer Inaktivierung der UNG, kontrolliert werden. UNG spaltet dabei Uracil aus allen uracilhaltigen DNAs, hat jedoch keinen Effekt auf natürliche (d.h. Ziel-)DNA. Die entstehenden abasischen Stellen blockieren die Replikation der DNA durch DNA-Polymerasen. Durch diese "carry-over prevention"-Technologie kann verhindert werden, dass PCR-Produkte aus resultierenden PCRs durch Kontamination zu falsch-positiven Resultaten führen können (Longo et al. (1990) Gene 93,125-128). Für diese Methode wird heute in der Regel eine UNG aus E. coli verwendet (WO 92/01814, EP 0 415 755). Auch die entsprechende Verwendung von UNGs für die isothermale Amplifikation ist beschrieben (EP 0 624 643).

Die meisten heute bekannten UNGs zeigen eine ausreichend hohe Spezifität für die effiziente Spaltung von Uracil aus Einzel- und Doppelstrang-DNA und sind somit prinzipiell zur Optimierung spezifischer Amplifikationsverfahren verwendbar. Die UNGs zeigen dagegen keine Aktivität gegenüber anderen, "normalen" DNA-Basen oder gegenüber Uracil in RNA.

Eine Reihe von UNGs, isoliert aus prokaryotischen und eukaryotischen Organismen sowie einige viralen Ursprungs, sind beschrieben. Mikrobielle UNGs sind insbesondere aus E. coli (T. Lindahl, PNAS 71 (9), 3649-3653 (1974); Lindahl et al., J. Biol. Chem. 252 (10), 3286-3294 (1977)), Bacillus subtilis (Cone et al., Biochemistry 16 (14), 3194-3201 (1977)), Bacillus stearothermophilus (Kaboev et al., FEBS Letters 132 (2), 337-340 (1981)), Thermothrix thiopara (Kaboev et al., J. Bacteriology 164 (1), 421-424 (1985)) und Micrococcus luteus (Leblanc et al., J. Biol. Chem. 257 (7), 3477-3483 (1982) bekannt. Darüber hinaus sind UNGs vom Menschen (Krokan et al., Nucl. Acid Res. 9 (11), 2599-2613 (1981); G. Seal et al., Biochim. Biophys. Acta 1097 (4) 299-308 (1991)) und einige UNGs viralen Ursprungs beschrieben. Des weiteren ist die strukturelle Basis für die Spezifität und Katalyse der UNG seit kurzem geklärt (Savva et al., Nature 373, 487-493 (1995); Mol et al., Cell 80, 869-878 (1995)).

Für die Anwendung für die "carry-over-prevention"-Methode im Rahmen von Amplifikationsverfahren, wie z.B. der PCR, genügen die meisten UNGs jedoch aufgrund ihres mangelnden Reinheitsgrads bzw. anderer Eigenschaften, insbesondere zu geringen Thermolabilität nicht den Anforderungen. So wird selbst nach drastischer Hitzeeinwirkung, wie beispielsweise 10 Minuten, 95°C und anschließender PCR eine Restaktivität an UNG nachgewiesen (Thornton et al., BioTechniques 13 (2), 180-183 (1992)). Die Restaktivität an UNG, d.h. der weitere Abbau von uracilhaltigen PCR-Produkten wird routinemäßig in der Regel dadurch verhindert, dass die entsprechenden Ansätze nach der PCR-Reaktion bei hohen Temperaturen von ca. 70° bis 72°C weiter inkubiert werden. Zudem wurde beobachtet, dass die Lagerung des PCR-Ansatzes/des PCR-Produkts selbst bei ca. 4°C oft zum weiteren Abbau des PCR-Produktes führt. Daher werden weit tiefere Temperaturen, wie ca. -20°C, für die Lagerung und/oder die Inhibierung der Restaktivität von UNG durch den Zusatz von Chloroform oder Phenol empfohlen. Darüber hinaus führte die Suche nach besser geeigneten hitzelabilen Mutanten bisher nicht zum Ziel (Duncan et al., J. Bacteriology 134, (3), 1039-1045 (1978); WO 92/01814). Die in WO 92/01814 beschriebenen Uracil-DNA-Glykosylasen aus verschiedenen Mikroorganismen zeichnen sich durchweg durch eine für PCR-Anwendungen zu geringe Thermolabilität aus. Diese benötigen insbesondere für den der eigentlichen Amplifikation vorgeschalteten Inkubationsschritt zur Erreichung einer möglichst geringen UNG-Reaktivität die doppelte Zeit im Vergleich zum thermolabilsten Enzym des Standes der Technik (E. coli UNG-Mutante, s.o. Duncan et al).

Somit läßt sich die Aktivität der derzeit zur Verfügung stehenden UNGs nicht vollständig bzw. nur unter Anwendung zusätzlicher; das gesamte Verfahren zusätzlich komplizierender Maßnahmen ausschalten.

Aufgabe der vorliegenden Erfindung war somit, ein thermolabiles Enzym mit Uracil-DNA-Glykosylase-Aktivität zur Verfügung zu stellen, durch welches die aus dem Stand der Technik bekannten Schwierigkeiten bei der Entfernung von Uracil aus DNA weitestgehend ausgeräumt bzw. vermieden werden können.

Die Aufgabe wird gelöst durch ein thermolabiles Enzym mit Uracil-DNA-Glykosylase-Aktivität, welches ein apparentes Molekulargewicht zwischen 23.000 und 24.000 aufweist, aus Mikroorganismen der Gattung Arthrobacter oder Micrococcus mit einem Reinheitsgrad von mindestens 95% (SDS-Gel) erhältlich ist und durch eine Halbwertszeit von weniger als 5 Minuten bei 40°C und ungefähr oder weniger als 2 Minuten bei 45°C charakterisiert ist. Neben Arthrobacter kommen hier insbesondere Mikroorganismen der Gattung Micrococcus in Betracht. Besonders vorteilhaft hat sich erwiesen, wenn der Mikroorganismus DSM 10239 (BMTU 3346) als Enzymquelle verwendet wird. DSM 10239 ist bei der Deutschen Sammlung für Mikroorganismen und Zellkulturen GmbH, Mascheroder Weg 1b, D-3 8124 Braunschweig hinterlegt.

Die Reinigung des erfindungsgemäßen Enzyms erfolgt in der Regel unterhalb von ca. 10°C, vorteilhafterweise bei ca. 4°C. Zunächst werden die Zellen durch dem Fachmann bekannte Maßnahmen aufgeschlossen; bevorzugt geschieht dies mechanisch mittels einer Hochdruckpresse oder eines Homogenisators. Anschließend werden die DNA-Bestandteile abgetrennt, z.B. durch eine Polymin-Fällung. Der Überstand wird zur weiteren Reinigung zunächst einer Hydroxyapatit-Chromatographie (z.B. Hydroxyapatit-Ultrogel) unterzogen, der eine Anionenaustauschchromatographie (bevorzugt an Q-Sepharose ff high load) und eine hydrophobe Interaktionschromatographie folgt. Letztere kann z.B. an Phenyl-Sepharose ff stattfinden.

Im Einzelnen wird für die Reinigung des Enzyms wie folgt vorgegangen:

Eine bestimmte Menge Zellen werden in Form ihres Trockengewichts in gefrorenem Zustand mit einer niedrig konzentrierten, im pH-Bereich von ca. 7,2 bis 8,0 gut puffernden Substanz, wie z.B. Phosphatpuffer mit einem SH-Reagenz suspendiert. Anschließend erfolgt zum Aufschluss der Zellen eine Inkubation mit Lysozym; in der Regel sind hier 30 Minuten bei ca. 4°C ausreichend. Der eigentliche Zellaufschluß erfolgt mechanisch, beispielsweise mittels einer Hochdruckpresse oder eines Homogenisators. In der Regel wird ein Aufschlussgrad von ca. 30 % erreicht.

Zur Abtrennung von Nukleinsäurebestandteilen werden diese unter nicht denaturierenden Bedingungen gefällt. Insbesondere hat sich hier eine stufenweise Fällung mit einer verdünnten Polymin-Lösung als geeignet erwiesen. Nach kurzer Inkubationsphase und Zentrifugation wird der Überstand vorteilhafterweise gegen die Pufferlösung dialysiert, die für die Suspensierung der Biomasse verwendet worden ist. Es hat sich gezeigt, daß die Dialyse in der Regel nach ca. 16 Stunden abgeschlossen ist. Das Dialysat wird über eine Hydroxyapatit-Ultrogelsäule aufgetrennt. In jedem Fall wird das entsprechende Chromatographie-Material zunächst mit der Lösung, in der sich auch die aufzutrennende Fraktion befindet, äquilibriert. Die das Enzym enthaltene Fraktion wird mit einem linearen Gradienten von ca. 10 mM bis 1 M Pufferlösung, z.B. eines Phosphatpuffers bei ca. pH 7,5 eluiert. Die vereinigten Fraktionen werden gegen eine bei ca. pH 8,0 puffernde Lösung dialysiert. Als Puffer ist hier beispielsweise Tris/HCl, aber auch Triethanolamine, N-Methyldiethanolamine oder andere organische bzw. anorganische Puffer mit einer Pufferkapazität zwischen pH 7,8 bis 8,4 geeignet. Das vereinigte Dialysat wird auf eine mit dem Dialysat-Puffer äquilibrierte Anionenaustauschersäule, wie beispielsweise Q-Sepharose ff high load aufgetragen und mit einem linearen Gradienten mit steigenden Konzentrationen Natriumchlorid eluiert. Die vereinigten Eluatfraktionen werden mit Ammoniumsulfat versetzt (Endkonzentration: 1,3 M) und auf ein hydrophobes Säulenmaterial aufgetragen. Als Säulenmaterial hat sich hier besonders Phenylsepharose ff als geeignet erwiesen. Das Säulenmaterial wird mit Puffer, beispielsweise Kaliumphosphat-Puffer enthaltend zusätzlich insbesondere ca. 1 M Ammoniumsulfat äquilibriert. Nach Beladung des Säulenmaterials wird mit einem linearen Gradienten enthaltend steigende Mengen an Glycerin bei pH ca. 6,0 eluiert. Die entsprechende Enzymaktivität aufweisenden Fraktionen werden vereinigt und gegen ein geeignetes Puffersystem, welches mindestens 100 mM Natriumchlorid und mindestens 40% Glycerin enthält dialysiert. Dieser Dialysepuffer hat sich gleichfalls als Lagerpuffer für das Enzym als geeignet erwiesen, wenn die Mischung aus ca. 10 bis 250 mM einer im schwach alkalischen pH-Bereich puffernder Substanz, wie beispielsweise Hepes, Tris oder Triethanolamin, ca. 0,1 bis 5 mM eines organischen Komplexbildners wie beispielsweise EDTA, eines SH-Gruppen stabilisierenden bzw. SS-Gruppen reduzierenden Agenzes in einer Konzentration von ca. 0,5 bis 5,0 mM, 200 bis 350 mM Natriumchlorid und ca. 45 bis 55% Glycerin aufweist. Als ganz besonders vorteilhaft für die Lagerung hat sich eine entsprechende Mischung erwiesen, die ca. 300 mM Natriumchlorid sowie ca. 50% (v/v) Glycerin sowie gegebenenfalls ca. 0,1 bis 5,0 mg/ml Rinderserumalbumin enthält. Das UNG-Enzym kann in einem solchen Puffer zwischen ca. +4° und -20°C bis zu einem Jahr aufbewahrt werden, ohne daß ein merklicher Akdvitätsvenust festzustellen ist.

Das Enzym kann mit dem erfindungsgemäßen Verfahren mit einem Reinheitsgrad von mindestens 95% (SDS-PAGE) erhalten werden. Das auf diese Weise isolierte Enzym besitzt mindestens eine spezifische Aktivität von 5x10⁴ Units/mg bei einem Temperaturoptimum von ca. 37 °C und einem pH-Optimum von ca. pH 6.5. Das apparente Molekulargewicht des Enzyms liegt zwischen 23000 und 24000, bei ca. 23400 dalton (SDS-PAGE).

Ein weiterer Vorteil des erfindungsgemäßen Enzyms ist, daß es nahezu frei von Fremdaktivitäten ist. D.h. es konnte gezeigt werden, daß bezogen auf die Gesamtaktivität der UNG weniger als 2%, in vielen Fällen weniger als 0,1% von fremden Fazymaktivitäten enthalten sind. Insbesondere Aktivitäten der folgenden Enzyme konnten nicht festgestellt werden: DNasen, Nicking Aktivität, Einzelstrang-DNasen, RNasen und Exonukleasen.

Ein weiterer Vorteil des erfindungsgemäßen UNG-Enzyms ist seine geringe Hitzebeständigkeit. Bei ca. 40°C beträgt die Halbwertszeit des Enzyms weniger als 5 Minuten, während einer Inkubation von ca. 45°C konnte eine Halbwertszeit von ungefähr 2 Minuten oder weniger, oft von ca. 60 Sekunden oder weniger ermittelt werden. Diese Stabilitätsdaten wurden in Tris/HCl-Puffer (pH-Bereich 8,3 bis 8,9), der darüber hinaus Magnesiumchlorid und Kaliumchlorid enthält, bestimmt.

Die erfindungsgemäße UNG ist zum Nachweis uracilhaltiger DNA bzw. zur Entfernung der Base Uracil aus DNA, insbesondere von uracilhaltigen PCR-Produkten geeignet. Die UNG wird hierfür in einem zwischen pH 7,5 und pH 9,2 puffernden System vorgelegt. Insbesondere haben sich hier solche Puffersysteme als geeignet erwiesen, die dem Fachmann für die Anwendung der PCR bekannt sind (Sambrook, J., Fritsch, E.F., Maniatis, T., Molecular Cloning: A Laboratory Manual, 2nd edition, Cold Spring Harbor Laboratory Press, 1989). Insbesondere haben sich hier anorganische oder organische Puffer, wie beispielsweise Tris-HCl in einem Konzentrationsbereich von 5 bis 100 mM, die zudem über 30 mM Kaliumchlorid und ca. 0,5 bis 3 mM Magnesiumchlorid enthalten, als vorteilhaft erwiesen. Die UNG liegt vorteilhafterweise in einer Konzentration von ca. 5 bis 40 U/ml, besonders bevorzugt von ca. 20 U/ml vor. Eine Inkubation von ca. eine bis 30 Minuten bei einer Temperatur von ca. 10°C bis 30°C hat sich in den meisten Fällen für den Abbau von kontaminierender uracilhaltiger DNA als ausreichend erwiesen. Anschließend wird die UNG inaktiviert, indem zwischen ca. 1 und 10 Minuten, vorteilhafterweise ca. 2 Minuten, auf ca. 95°C erhitzt wird. Als vorteilhaft hat sich dabei erwiesen, daß die erfindungsgemäße UNG nach der Inaktivierung bei längerer Inkubation (ca. 4°C) von mehreren Stunden (ca. 4h) keine Restaktivität aufweist. Diese Eigenschaft hat sich als besonders vorteilhaft in der sogenannten "carry-over-prevention"-Methode erwiesen, da die bekannten UNGs, wie z.B. das aus E. coli erhältliche Enzym, weniger leicht durch Hitzeeinwirkung zu inaktivieren sind und somit eine deutlich höhere Restaktivität zurück bleibt. Durch die Anwesenheit einer geringeren Restaktivität nach der Behandlung von DNA, beispielsweise PCR-Produkten, mit dem erfindungsgemäßen Enzym ist zudem der Vorteil der besseren bzw. längeren Lagerbarkeit verbunden.

Ein weiterer Gegenstand der Erfindung ist ein Kit (Reagenz) zur Vervielfältigung spezifischer Nukleinsäurefragmente, insbesondere zur Durchführung von PCR unter den beschriebenen verbesserten carry-over-prevention-Bedingungen. Der Kit enthält neben den konventionellen Nukleotidtriphosphaten das Nukleotidtriphosphat dUTP, eine thermostabile Polymerase, die erfindungsgemäße hitzelabile UNG sowie einen geeigneten Reaktionspuffer. Im besonderen enthält dieser Kit die hitzelabile UNG, in einer Konzentration von 0,1 bis 5 U/µl. Daneben enthält der Kit die Nukleotidtriphosphate dATP, dCTP, dGTP in einer Konzentration von 10 mM sowie das Nukleotidtriphosphat dUTP in einer Konzentration von 30 mM. Des weiteren enthält der Kit einen Puffer zur Durchführung der Dekontamination, Hitzeinaktivierung der UNG und PCR. Dieser Puffer ist im schwach-alkalischen Bereich gepuffert, zwischen pH 7,5 und 9,2, vorzugsweise pH 8,3 bis 8,9. Geeignete Puffersubstanzen sind dabei beispielsweise Tris/HCl 10 mM. Ferner enthält der Puffer ca. 10 bis 100 mM KCI (bevorzugt sind 50 mM), MgCl₂ zwischen 1,0 und 5 mM. Die bevorzugt verwendete Polymerase ist Taq-DNA-Polymerase, isoliert aus Thermus aquaticus; die Konzentration beträgt 2 bis 10 U/µl, bevorzugt 5 U/µl.

Zusammenfassend kann somit festgehalten werden, daß das erfindungsgemäße hitzelabile Enzym mit Uracil-DNA-Glykosylase-Aktivität im Vergleich zu bekannten Enzymen überraschenderweise leichter durch Hitzebehandlung zu inaktivieren ist. Zudem konnte gezeigt werden, daß die Verwendung des erfindungsgemäBen Enzyms bei der "carryover-prevention"-Methode eine deutlich geringere Restaktivität nach Durchführung der PCR zeigt. Dies führt zu einer deutlichen Verbesserung bezüglich Quantität und Qualität des PCR-Produktes; insbesondere da uracilhaltige PCR-Produkte nach der PCR nicht aufgrund von Restaktivität (und/oder Reaktivierung) der UNG degradiert werden.

### Erläuterungen der Abbildungen:

### Abbildung 1:

### Vergleich Hitzeinaktivierung der UNG aus DSM 10239 und E. coli.

Es wurden jeweils 1 U der UNG aus DSM 10239 und E. coli in 100 µl PCR-Puffer verdünnt und bei 40 und 45°C inkubiert. Zu bestimmten Zeiten wurden Proben genommen und die verbleibende Restaktivität bestimmt,
(-▲- (40°C), -■-(45°C): UNG DSM 10239;
-Δ- (44°C), -□- (45°C): UNG E. coli).

### Abbildung 2A:

Bestimmung der Restaktivität von UNG nach Inaktivierung und PCR.
Es wurden jeweils 2 U der UNG aus DSM 10239 sowie aus E. coli zu einem PCR-Ansatz zugegeben. Danach wurde die UNG für 2 min bei 95°C inaktiviert Anschließend wurde die PCR (Amplifikation mit einem 103 Basenpaare langen Fragment) durchgeführt Nach der PCR wurde die Probe auf 4°C abgekühlt. Die Spuren A 1-4 zeigen den Versuch des Ansatzes für die UNG aus DSM 10239. Die Spuren B 1-4 zeigen den entsprechenden Versuch für die UNG aus E. coli, die Spuren C 1-4 die Kontrollansätze ohne UNG. Die Spuren A1, B1, C1 zeigen die Probe für eine Inkubationszeit T = 0; die Spuren A2, B2, C2 die Probe nach einer Inkubationszeit T = 1 h; die Spuren A3, B3, C3 nach 4 h Inkubation und die Spuren A4, B4, C4 nach 16 h Inkubation. Nach 16 h zeigen sich bei beiden UNGs Abbauprodukte des PCR-Produktes. Bei der UNG aus E. coli ist das Auftreten solcher Abbauprodukte bereits zur Zeit T = 0 zu beobachten. Die Spuren D und E zeigen den Verlauf des Abbaus der PCR-Produkte bei Zugabe der UNG aus DSM 10239 (Spur D) und E. coli (Spur E) nach der PCR.

### Abbildung 2B:

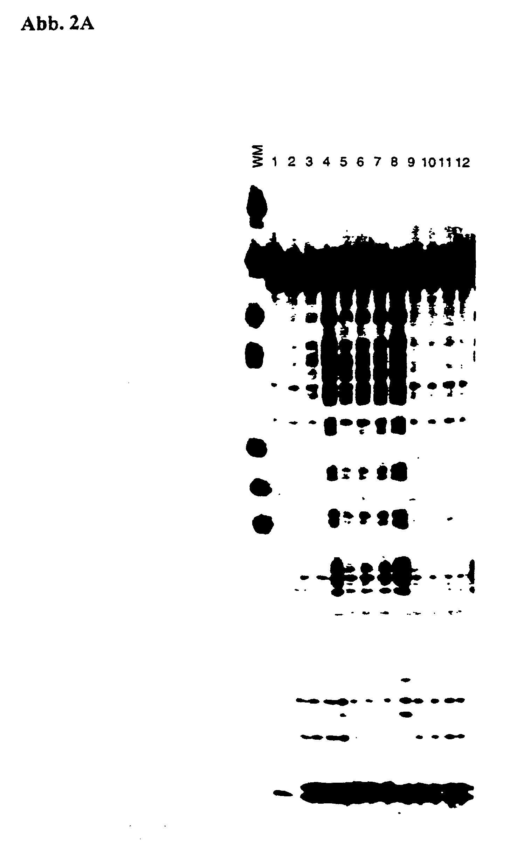

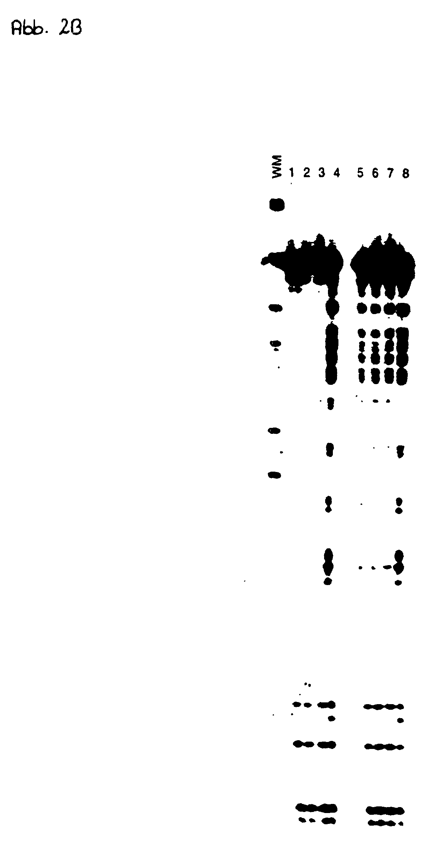

Versuchsanordnung wie in Abb. 2A; die Inaktivierungszeit der UNG betrug jedoch 10 min bei 95°C. Die Spuren A, B entsprechen den Spuren A, B der Abb. 2A. Es ist deutlich zu erkennen, daß die UNG aus DSM 10239 im Bereich zwischen T = 0 und T = 4 h keinerlei Abbauprodukte des PCR-Fragmentes aufweist.

Die folgenden Beispiele erläutern die Erfindung weiter:

### Beispiel 1:

### Reinigung der hitzelabilen Uracil-DNA-Glykosylase

### Definition der Enzymeinheiten:

1 U ist definiert als die Menge von Uracil-DNA-Glykosylase, die benötigt wird, um 1 µg Einzelstrang uracilhaltige DNA (Bakteriophage M13, gewachsen in E. coli CJ236 DUT negativ, UNG negativ) bei 37°C in 60 min vollständig abzubauen.

Testvolumen: 50 µl, Konzentration 60 mM Tris/HCl, pH 8/,0; 1 mM EDTA, 1 mM DTT, 0,1 mg/ml BSA.

Nach Inkubation für 60 min bei 37°C werden 16,5 µl 0,6 M NaOH zugegeben, für 5 min bei 37°C inkubiert, danach auf Eis abgestoppt und anschließend 16,5 µl 0,6 M HCl zugegeben. Die Auswertung erfolgt auf einem 1% igen Agarosegel.

### Reinigung:

Die Reinigung der Uracil-DNA-Glykosylase erfolgt bei 4°C. Das hier beschriebene Verfahren bezieht sich auf die Reinigung der Uracil-Glykosylase aus DSM 10239. Das Reinigungsverfahren umfaßt folgende Schritte:

Aufschluß der Zellen in einer Hochdruckpresse, Polymin-Fällung zur Abtrennung der DNA, Reinigung der UNG durch Chromatographie an HA-Ultrogel, Anionenaustauschchromatographie (Q-Sepharose ff high load) und hydrophobe Interaktionschromatographie (Phenyl-Sepharose ff).

### Lösungen:

- Puffer 1:: 10 mM Kaliumphosphat, pH 7,5, 1 mM β-Mercaptoethanol
- Puffer 2:: 10 mM Tris/HCl, pH 8,0/4°C, 1 mM β-Mercaptosthanol
- Puffer 3:: 100 mM Kaliumphosphat, pH 6,0, 1 M Ammoniumsulfat, 1 mM β-Mercaptoethanol
- Puffer 4:: 100 mM Kaliumphosphat, pH 6,0, 10% Glycerin, 1 mM β-Mercaptoethanol
- Lagerpuffer:: 50 mM Hepes/KOH, pH 8,0, 1 mM EDTA, 1 mM DTT, 300 mM NaCl, 50% Glycerin

40 g Biomasse (Trockengewicht) werden mit 400 ml Puffer 1 versetzt, aufgetaut und suspendiert. Die Suspension wird mit 100 mg Lysozym versetzt und für 30 min bei 4°C gerührt. Anschließend erfolgt der Aufschluß der Zellen in einer Hochdruckpresse in zwei Durchgängen. Der Druck beträgt dabei 550 kg/cm². Der Aufschlußgrad beträgt unter diesen Bedingungen üblicherweise 20-30%.

Anschließend erfolgt eine Polyminfällung: 10 ml 10%ige Polymin-P-Lösung werden tropfenweise zugegeben. Falls die Fällung nicht vollständig ist, erfolgt eine weitere Polymin-Zugabe in je 2 ml-Schritten. Nach beendeter Titration wird das Präzipitat ca. 30 min bei 4°C stehen gelassen. Anschließend wird die Suspension für 30 min bei 13.000 x g bei 4°C zentrifugiert. Der Überstand der Zentrifugation wird insgesamt gegen 5x 5 Liter Puffer 1 dialysiert (Dauer 16 h). Das Dialysat wird auf eine mit Puffer 1 äquilibrierte HA-Ultrogelsäule (2,6 x 10 cm) aufgezogen und mit ca. 500 ml Puffer 1 gewaschen. Anschließend wird das Enzym mit einem linearen Gradienten aus Puffer 1 und Puffer 1 + 1 M Kaliumphosphat pH 7,5 in einem Gesamtvolumen von 1,5 l eluiert.

Die Fließgeschwindigkeit beträgt 5 ml pro Minute, die Fraktionsgröße 10 ml pro Fraktion.

Das Enzym eluiert zwischen 50 und 150 mM Kaliumphosphat. Die gepoolten Lösungen werden gegen 4 x 2 Liter Puffer 2 dialyisert. Die dialysierte Lösung wird auf eine mit Puffer 2 äquilibrierte Q-Sepharose ff-high load (2,6 x 10 cm) aufgezogen und mit ca. 500 ml Puffer 2 die Säule gewaschen.

Anschließend wir das Enzym mit einem linearen Gradienten aus Puffer 2 und Puffer 2 + 1 M NaCl in einem Gesamtvolumen von 1,5 Liter eluiert. Fließgeschwindigkeit beträgt ca. 10,0 ml/min, die Fraktionsgröße 10 ml.

Das Enzym eluiert zwischen 200 und 300 mM NaCl-Konzentration.

Zu den gepoolten Fraktionen wird festes Ammoniumsulfat bis zu einer Endkonzentration von 1,3 M unter Rühren bei 4°C zugegeben und gelöst. Diese Lösung wird auf eine mit Puffer 3 äquilibrierte Phenylsepharose ff-Säule (1,6 x 10 cm) geladen. Nach Waschen mit ca. 200 ml Puffer 3 wird das Enzym mit einem linearen Gradienten aus Puffer 3 und Puffer 4 in einem Volumen von 100 ml eluiert. Die Fließgeschwindigkeit beträgt ca. 2,5 ml pro min, die Fraktionsgröße 4 ml. Die aktiven Fraktionen werden vereinigt und gegen Lagerpuffer dialysiert. Die gereinigte UNG ist zwischen +4°C und -20°C in Lagerpuffer stabil.

Die beschriebene Methode liefert eine ca. 23,4 kda große Uracil-DNA-Glykosylase mit einem Reinheitsgrad von mindestens 95 % (8-25% SDS-PAGE, Phastgel von Pharmacia, Phastgelsystem) und einer spezifischen Aktivität von mindestens 5 x 10⁴ Units/mg (Proteinbestimmung nach Coomassie). Das Enzym ist zudem frei von kontaminierenden Fremdaktivitäten (Nicking-Aktivität, Exonuklease und Endonuklease).

### Bestimmung von kontaminierenden Fremdaktivitäten:

Der Test auf das Vorhandensein von kontaminierenden Fremdenzymaktivitäten wurde in einer Lösung bestehend aus 10 mM Tris-HCl, pH 7,5, 10 mM MgCl₂, 1 mM DTE durchgeführt. Die einzelnen Enzymfraktionen (20 µl) wurden mit den entsprechenden Nukleinsäuren inkubiert. Sogenannte Nicking-Aktivität wurde durch Inkubation von 1 µg pBR322 für 16 Stunden bei 37°C bestimmt. Einzel- und doppelsträngige Nukleasen wurden unter Verwendung von M13mp9-ss DNA und entsprechend von λ/Eco RI, HindII getestet; die Inkubation erfolgte bei 37°C für 16 Stunden. Die Abwesenheit von RNasen wurde getestet durch Inkubieren der Proben mit 5 µg MS 2 RNA für 1 Stunde bei 37°C. Für den Test auf Exonukleasen wurden die Proben mit 1 µg [3H]-gelabelter DNA 4 Stunden bei 37°C inkubiert und die freigesetzten [3H]-markierten Nukleotide bestimmt.

### Beispiel 2:

### Hitzelabilität der UNG

Die Hitzelabiltät (Hitzeinaktivierbarkeit) der UNG aus DSM 10239 wurde mit einem radioaktiven Testsystem bestimmt.

Zu diesem Zweck wurde ein radioaktives Testsubstrat über Random primed labeling hergestellt. 5 ml Reaktionsvolumen enthielten: 2,5 mg Kalbsthymus-DNA, je 0,5 µM dCTP, dATP, dGTP, 2,6 nM H3-dUTP, 23 nM dUTP, 1 ml Hacanukleotidmix (62,5 OD/ml) und 5 KU Klenow-Fragment. Der Reaktionsansatz wurde 1 h bei 37°C inkubiert. Nicht eingebaute Nukleotide wurden durch Chromatographie an Sephadex G50 (2,5 x 10 cm) abgetrennt (Sephadex G50, äquilibriert in 10 mM Tris/HCl, pH 8,0/4°C). Die Fraktionen, die markierte DNA enthielten, wurden gesammelt und durch Lyophilisation eingeengt.

### Durchführung des Testes:

Der Testansatz (50 µl) enthielt 5 µl der markierten Kalbsthymus-DNA (10.000 cpm entspricht 0,82 pMol H3-Uracil), 60 mM Tris/HCl, pH 8,0, 1 mM EDTA, 1 mM DTT, 0,1 mg/ml BSA. Nach Zugabe von UNG in einer geeigneten Verdünnung wurde der Reaktionsansatz für 10 min bei 37°C inkubiert.

Anschließend wurde auf Eis abgestoppt, 100 µl Fällungs-DNA (1mg/ml) zugegeben und 300 µl 10%ige Trichloressigsäure (TCA) zugegeben. Nach 10 min Inkubation auf Eis (4°C) wurde für 5 min in einer Tischzentrifuge zentrifugiert, 400 µl des Überstandes wurden zur Zählung in einem Szintillationszähler verwendet.

Im Rahmen der "carry-over-prevention"-Methode wird UNG in einem für PCR geeigneten Puffer verwendet. Die Inaktivierungskinetik der erfindungsgemäßen UNG wurde deshalb in einem für PCR geeigneten Puffer durchgeführt. Der PCR-Puffer enthielt 10 mM Tris/HCl, pH 8,3, 1,5 mM MgCl₂, 50 mM KCI.

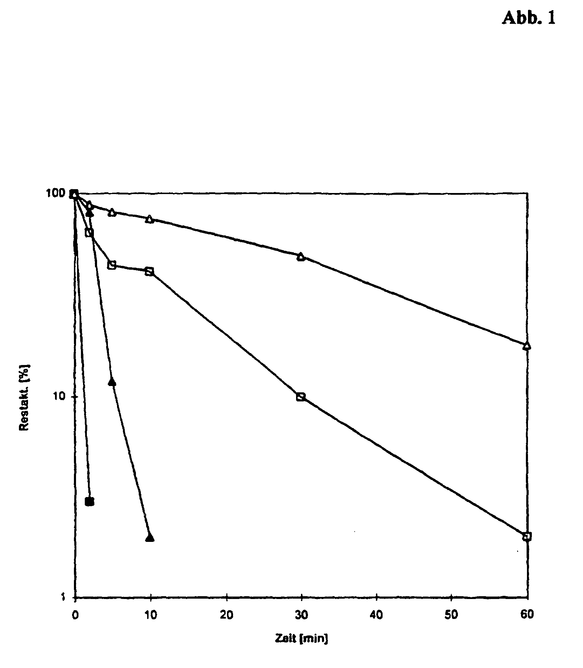

1 U UNG wurde in 100 µl PCR-Puffer verdünnt und bei unterschiedlichen Temperaturen inkubiert. Zu bestimmten Zeiten wurden Proben genommen und die verbleibende Restaktivität mit dem oben beschriebenen Testsystem bestimmt (Tabelle 1). Dabei wurden für die erfindungsgemäße UNG folgende Halbwertszeiten ermittelt: 0,5 min bei 45°C, 2 min bei 40°C (Abb. 1). Für die UNG aus E. coli wurden unter entsprechenden Bedingungen folgende Halbwertszeiten ermittelt: 8 min bei 45°C und 27 min bei 40°C.

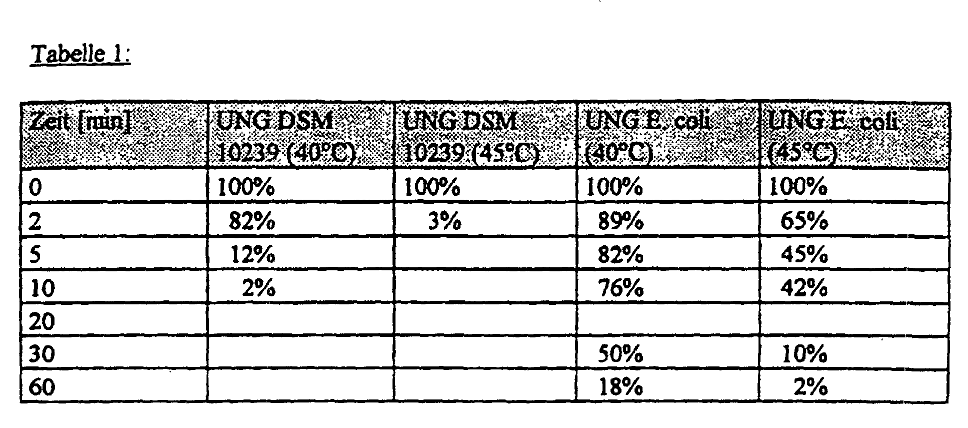

### Beispiel 3 :

### Restaktivität der erfindungsgemäßen UNG nach Inaktivierung und PCR

Das im folgenden beschriebene System dient zum Nachweis von Restaktivitäten an UNG nach Hitzeinaktivierung und anschließender PCR Dabei wird der Abbau eines uracilhaltigen PCR-Produkts verfolgt. Die Detektion des PCR-Produkts erfolgt über den Nachweis einer eingebauten Digoxigenin (DIG)-Markierung. Diese Markierung wurde durch Verwendung eines am 5'-Ende DIG-markierten Primers in der PCR vorgenommen; der zweite Primer trägt keine Markierung. Der Abbau des uracilhaltigen PCR-Produkts erfolgt durch den Nachweis der Abbauprodukte. Die Abbauprodukte werden dabei über ein Sequenzgel aufgetrennt und die DIG-Markierung über ein Anti-DIG/Chemoluminescenz-System detektiert.

Es wurde eine 103 Basenpaare lange Sequenz aus dem Multiple Cloning Site des pUC 18-Vectors amplifiziert. Als Primer wurden dazu der pUC-sequencing, 5'-Digoxigeninmarkierte Primer (Sequenz: 5'-DIG-d[GTAAAACGA CGGCCAGT]-3' sowie der pUCreverse sequencing primer (Sequenz: d[CAGGAAAC AGCTATGAC]-3' verwendet. 100 µl des Ansatzes enthielten PCR-Puffer mit 10 mM Tris/CCl, pH 8,3, 50 mM KCl, 1,5 mM MgCl₂; je 200 µM der Nukleotide dATP, dCTP, dGTP sowie 600 µM dUTP, 2,5 U Taq-DNA-Polymerase, 1 ng pUC 18-DNA/Pst 1, 2 U UNG sowie je 1 µM der beiden Primer. Danach werden die Proben in einen Thermocycler (z.B. Perkin Elmer 9600) überführt. Die Hitzeinaktivierung der UNG erfolgt innerhalb von 2-10 min bei 95°C. Anschließend wird die PCR zur Amplifikation des 103 Basenpaare-Fragments durchgeführt. Die PCR besteht aus 25 Zyklen á 1 min bei 94°C, 1min bei 50°C sowie 3 min bei 72°C. Danach wird die Probe bei 4°C aufbewahrt und zu geeigneten Zeitpunkten Proben entnommen, um den Abbau des PCR-Produkts nachzuweisen. 20 µl der entsprechenden Probe werden mit 5 µl 0,6 M NaOH versetzt, für 5 min bei 37°C inkubiert, danach auf Eis abgestoppt und anschließend 5 µl 0,6 M HCl sowie 4 µl Formamid-Stopper-Lösung zugegeben. Anschließend werden die Proben 3 min bei 95°C erhitzt, um Einzelstränge zu erhalten.

1,5 µl der Probe werden auf ein 8%iges Sequenzgel aufgetragen und getrennt (Laufzeit 50 min bei 2500 V, 26 mA). Die anschließende Detektion des DIG-markierten PCR-Produkts sowie der DIG-markierten Abbauprodukte erfolgte nach dem Protokoll des DIG Taq DNA Sequencing Kit (Boehringer Mannheim) und des DIG-Lumineszenz-Detektion-Kit (Boehringer Mannheim).

Abb. 2A zeigt die Auswertung eines solchen Experiments. Dabei wurden die PCR-Ansätze bei 4°C aufgewahrt und nach 0 h, 1 h, 4 h, 16 h jeweils Proben entnommen.

Im Vergleich zu der UNG aus DSM 10239 (Spur A 1-4) wurde die UNG aus E. coli (Spur B 1-4) eingesetzt. Als Kontrolle diente ein Ansatz ohne UNG (Spur C 1-4). Im Gegensatz zur E. coli-UNG treten bei der erfindungsgemäßen UNG in den Zeiten 0 bis 4 h nahezu keine Abbauprodukte auf. Bei einer Hitzeinaktivierung für 2 Minuten bei 95°C zeigt die erfindungsgemäße UNG somit einen deutlichen Vorteil gegenüber der UNG aus E. coli (Abb. 2A). Die minimale verbleibende Aktivität der erfindungsgemäßen UNG kann durch eine Verlängerung des Inaktivierungsschrittes (z.B. 10 min bei 95°C) weiter gesenkt werden (Abb. 2B).

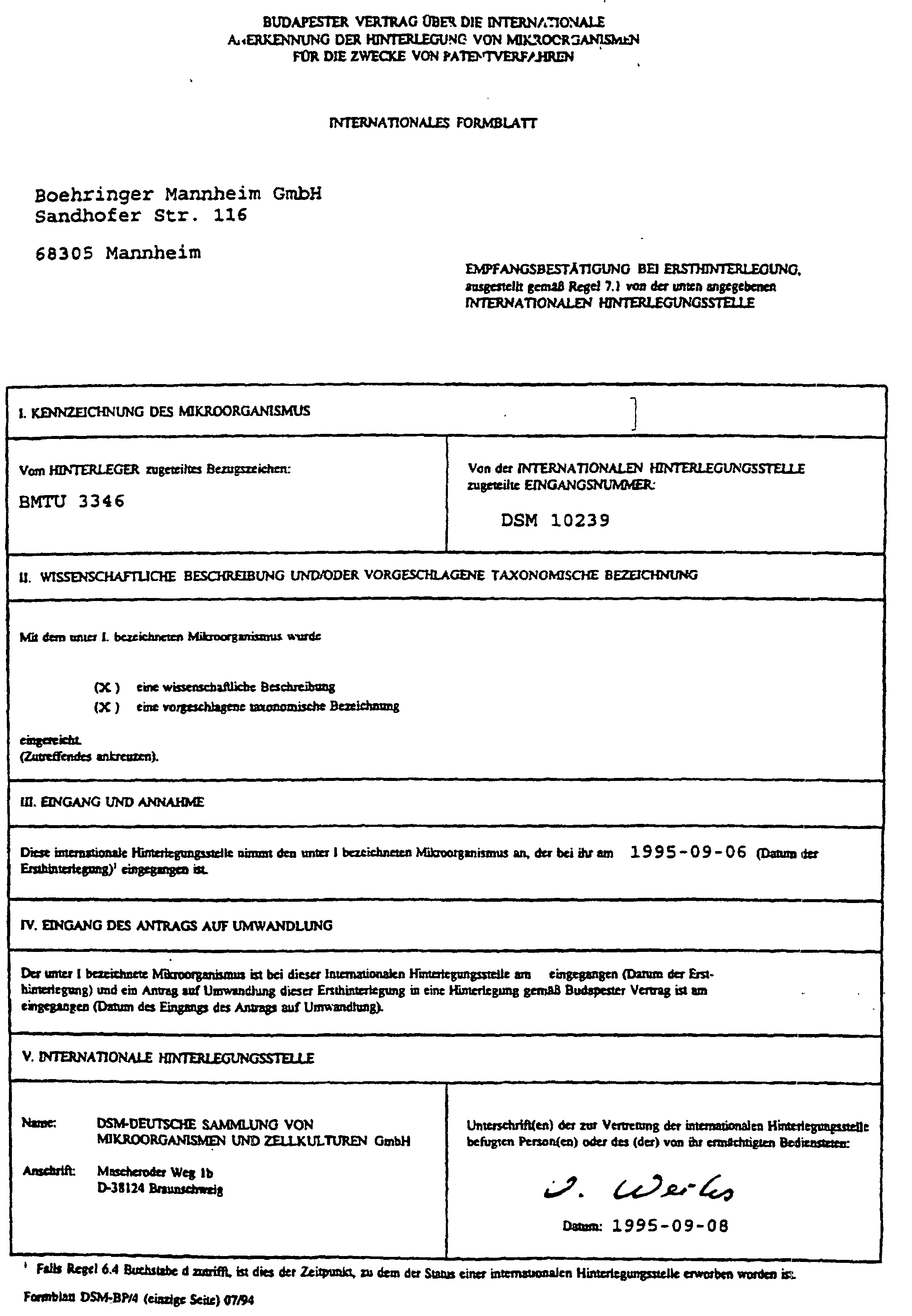
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

INTERNATIONALES FORMBLATT

Boehringer Mannheim GmbH
Sandhofer Str. 116

68305 Mannheim

EMPFANGSBESTÄTIGUNG BEI ERSTHINTERLEGUNG,
ausgestellt gemäß Regel 7.1 von der unten angegebenen
INTERNATIONALEN HINTERLEGUNGSSTELLE

I. KENNZEICHNUNG DES MIKROORGANISMUS

Vom HINTERLEGER zugeteiltes Bezugszeichen: | Von der INTERNATIONALEN HINTERLEGUNGSSTELLE zugeteilte EINGANGSNUMMER:
BMTU 3346 | DSM 10239

II. WISSENSCHAFTLICHE BESCHREIBUNG UND/ODER VORGESCHLAGENE TAXONOMISCHE BEZEICHNUNG

Mit dem unter I. bezeichneten Mikroorganismus wurde

(X) eine wissenschaftliche Beschreibung
(X) eine vorgeschlagene taxonomische Bezeichnung

eingereicht.
(Zutreffendes ankreuzen).

III. EINGANG UND ANNAHME

Diese internationale Hinterlegungsstelle nimmt den unter I bezeichneten Mikroorganismus an, der bei ihr am 1995-09-06 (Datum der
Ersthinterlegung)¹ eingegangen ist.

IV. EINGANG DES ANTRAGS AUF UMWANDLUNG

Der unter I bezeichnete Mikroorganismus ist bei dieser Internationalen Hinterlegungsstelle am eingegangen (Datum der Erst-
hinterlegung) und ein Antrag auf Umwandlung dieser Ersthinterlegung in eine Hinterlegung gemäß Budapester Vertrag ist am
eingegangen (Datum des Eingangs des Antrags auf Umwandlung).

V. INTERNATIONALE HINTERLEGUNGSSTELLE

Name: DSM-DEUTSCHE SAMMLUNG VON
MIKROORGANISMEN UND ZELLKULTUREN GmbH

Anschrift: Mascheroder Weg 1b
D-38124 Braunschweig | Unterschrift(en) der zur Vertretung der internationalen Hinterlegungsstelle befugten Person(en) oder des (der) von ihr ermächtigten Bediensteten:


Datum: 1995-09-08

¹ Falls Regel 6.4 Buchstabe d zutrifft, ist dies der Zeitpunkt, zu dem der Status einer internationalen Hinterlegungsstelle erworben worden ist.

Formblatt DSM-BP/4 (einzige Seite) 07/94

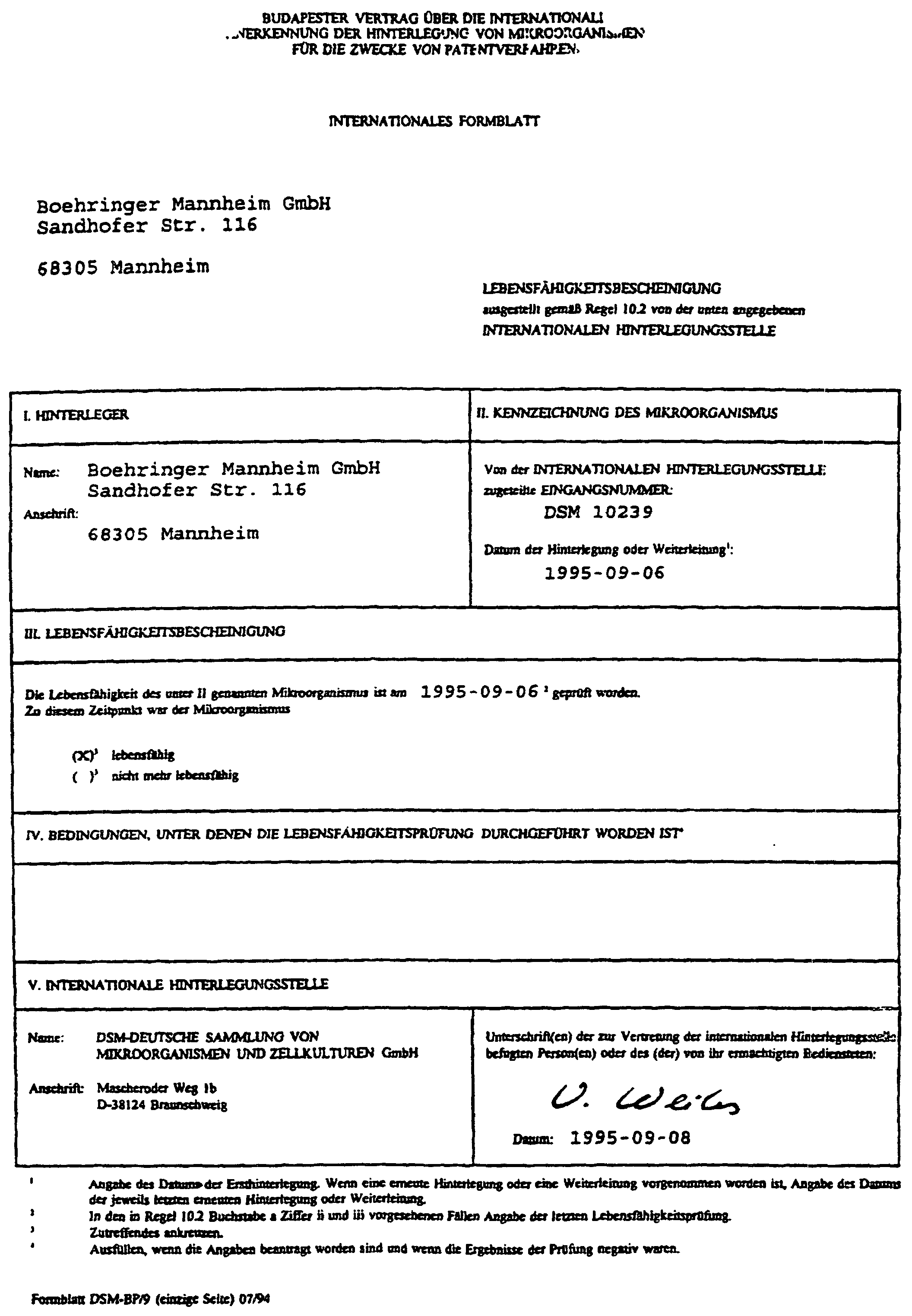
BUDAPESTER VERTRAG ÜBER DIE INTERNATIONAL[?]
[?][?]NERKENNUNG DER HINTERLEGUNG VON MIKROORGANISMEN
FÜR DIE ZWECKE VON PATENTVERFAHREN

INTERNATIONALES FORMBLATT

Boehringer Mannheim GmbH
Sandhofer Str. 116

68305 Mannheim

LEBENSFÄHIGKEITSBESCHEINIGUNG
ausgestellt gemäß Regel 10.2 von der unten angegebenen
INTERNATIONALEN HINTERLEGUNGSSTELLE

I. HINTERLEGER | II. KENNZEICHNUNG DES MIKROORGANISMUS
Name: Boehringer Mannheim GmbH
Sandhofer Str. 116
Anschrift:
68305 Mannheim | Von der INTERNATIONALEN HINTERLEGUNGSSTELLE zugeteilte EINGANGSNUMMER:
DSM 10239
Datum der Hinterlegung oder Weiterleitung¹:
1995-09-06

III. LEBENSFÄHIGKEITSBESCHEINIGUNG

Die Lebensfähigkeit des unter II genannten Mikroorganismus ist am 1995-09-06 ² geprüft worden.
Zu diesem Zeitpunkt war der Mikroorganismus

(X)³ lebensfähig
( )³ nicht mehr lebensfähig

IV. BEDINGUNGEN, UNTER DENEN DIE LEBENSFÄHIGKEITSPRÜFUNG DURCHGEFÜHRT WORDEN IST⁴

V. INTERNATIONALE HINTERLEGUNGSSTELLE

Name: DSM-DEUTSCHE SAMMLUNG VON
MIKROORGANISMEN UND ZELLKULTUREN GmbH

Anschrift: Mascheroder Weg 1b
D-38124 Braunschweig | Unterschrift(en) der zur Vertretung der internationalen Hinterlegungsstelle befugten Person(en) oder des (der) von ihr ermächtigten Bediensteten:
Datum: 1995-09-08

¹ Angabe des Datums der Ersthinterlegung. Wenn eine erneute Hinterlegung oder eine Weiterleitung vorgenommen worden ist, Angabe des Datums der jeweils letzten erneuten Hinterlegung oder Weiterleitung.
² In den in Regel 10.2 Buchstabe a Ziffer ii und iii vorgesehenen Fällen Angabe der letzten Lebensfähigkeitsprüfung.
³ Zutreffendes ankreuzen.
⁴ Ausfüllen, wenn die Angaben beantragt worden sind und wenn die Ergebnisse der Prüfung negativ waren.

Formblatt DSM-BP/9 (einzige Seite) 07/94

## Patentansprüche

1. Thermolabiles Enzym mit Uracil-DNA-Glykosylase-Aktivität, welches ein apparates Molekulargewicht zwischen 23.000 und 24.000 Dalton aufweist, erhältlich aus Mikroorganismen der Gattung Arthrobacter oder Micrococcus mit einem Reinheitsgrad von mindestens 95% (SDS-Gel) und Halbwertszeiten von weniger als 5 Minuten bei 40°C und weniger als 2 Minuten bei 45°C.

2. Enzym nach Anspruch 1, **dadurch gekennzeichnet, dass** der Gehalt an kontaminierenden Fremdaktivitäten kleiner als 2 % ist und die spezifische Aktivität mindestens 5 x 10⁴ U/mg Protein beträgt.

3. Enzym nach Anspruch 1 oder 2, **dadurch gekennzeichnet, dass** das Temperaturoptimum 37°C und das pH-Optimum 6,5 beträgt.

4. Enzym nach einem der Ansprüche 1, 2 oder 3, **dadurch gekennzeichnet, dass** das Enzym aus dem Stamm DSM 10239 isoliert wird.

5. Stabilisierte Lösung des thermolabilen Enzyms mit Uracil-DNA-Glykosylase-Aktivität nach Anspruch 1 bis 4, **dadurch gekennzeichnet, dass** das Enzym in einer Mischung bestehend aus 10 bis 250 mM einer im schwach alkalischen Bereich puffernder Substanz, 0,1 bis 5 mM eines Komplexbildners, 0,5 bis 5 mM eines SH-Gruppen stabilisierenden Agenzes, mindestens 100 mM Natriumchlorid, 45 bis 55 % (v/v) Glycerin und gegebenenfalls 0,1 bis 5,0 mg/ml Rinderserumalbumin enthalten ist.

6. Stabilisierte Enzymlösung nach Anspruch 5, **dadurch gekennzeichnet, dass** 50 mM Hepes/KOH, pH 8,0, 1 mM EDTA, 1 mM Dithiothreithol, 300 mM Natriumchlorid und 50 % (v/v) Glycerin enthalten sind.

7. Verfahren zur Gewinnung eines Enzyms nach einem der Ansprüche 1 bis 4 aus Mikroorganismen der Gattung Arthrobacter oder Micrococcus, **dadurch gekennzeichnet, dass** nach Aufschluss der Zellen und Abtrennung von DNA eine Hydroxyapatit-Chromatographie, eine Anionenaustauschchromatographie und eine hydrophobe Chromatographie ausgeführt wird.

8. Verfahren nach Anspruch 7, **dadurch gekennzeichnet, dass** die DNA-Abtrennung durch Polymin-Fällung erfolgt.

9. Verfahren nach Anspruch 7 oder 8, **dadurch gekennzeichnet, dass** die Anionenaustauschchromatographie an Q-Sepharose ff (high load) erfolgt.

10. Verfahren nach Anspruch 7, 8 oder 9, **dadurch gekennzeichnet, dass** der hydrophobe Chromatographieschritt an Phenyl-Sepharose ff erfolgt.

11. Verfahren nach Anspruch 7, 8, 9 oder 10, **dadurch gekennzeichnet, dass** der Mikroorganismus DSM 10239 verwendet wird.

12. Verwendung eines thermolabilen Enzyms mit Uracil-DNA-Glykosylase-Aktivität nach einem der Ansprüche 1 bis 6 zum Nachweis uracilhaltiger DNA bzw. zur Entfernung der Base Uracil aus DNA, **dadurch gekennzeichnet, dass** eine Mischung mit uracilhaltiger DNA mit dem Enzym eine Minute bis 30 Minuten bei einer Temperatur von 10°C bis 30°C inkubiert und 1 bis 10 Minuten auf 95°C erhitzt wird.

13. Verwendung nach Anspruch 12, **dadurch gekennzeichnet, dass** es sich bei der uracilhaltigen DNA um spezifisch amplifizierte DNA handelt.

14. Verwendung nach Anspruch 12 oder 13, **dadurch gekennzeichnet, dass** bei Inkubation zwischen 30 Sekunden und vier Stunden bei 4°C keine Aktivität von Uracil-DNA-Glykosylase mehr feststellbar ist.

15. Kit zur Vervielfältigung von spezifischen Nukleinsäuren bzw. entsprechender Fragmente, **dadurch gekennzeichnet, dass** folgende Komponenten enthalten sind:
(a) ein thermolabiles Enzym gemäß einem der Ansprüche 1 bis 4 in einem geeigneten Lagerpuffer,
(b) die Nukleotidtriphosphate dATP, dCTP, dGTP und dUTP,
(c) eine thermostabile DNA-Polymerase und
(d) eine im pH-Bereich von 7,5 bis 9,2 puffernde Substanz (Reaktionspuffer).

16. Kit nach Anspruch 15, **dadurch gekennzeichnet, dass** das thermolabile Enzym in einer Konzentration von 0,1 bis 5,0 U/µl vorliegt.

17. Kit nach Anspruch 15 oder 16, **dadurch gekennzeichnet, dass** die Nukleotidtriphosphate dATP, dCTP und dGTP jeweils ein Drittel der Konzentration von dUTP ausmachen.

18. Kit nach einem der Ansprüche 15 bis 17, **dadurch gekennzeichnet, dass** 2 bis 10 U/µl (Endkonzentration) der thermostabilen DNA-Polymerase vorliegt.

19. Kit nach einem der Ansprüche 15 bis 18, **dadurch gekennzeichnet, dass** der Reaktionspuffer außerdem 10 bis 100 mM Kaliumchlorid und/oder 1,0 bis 5,0 mM Magnesiumchlorid enthält.

## Claims

1. Thermolabile enzyme with uracil-DNA-glycosylase activity which has an apparent molecular weight between 23000 and 24000 Daltons obtainable from gram-positive microorganisms of the genus Arthrobacter or Micrococcus having a degree of purity of at least 95 % (SDS gel) and half-lives of less than 5 minutes at 40°C and of less than 2 minutes at 45°C.

2. Enzyme as claimed in claim 1, wherein the content of contaminating foreign activities is less than 2 % and the specific activity is at least 5 x 10⁴ U/mg protein.

3. Enzyme as claimed in claim 1 or 2, wherein the temperature optimum is 37°C and the pH optimum is 6.5.

4. Enzyme as claimed in one of the claims 1, 2 or 3, wherein the enzyme is isolated from the strain DSM 10239.

5. Stabilized solution of the thermolabile enzyme with uracil-DNA-glycosylase activity as claimed in claim 1 to 4, wherein the enzyme is contained in a mixture composed of 10 to 250 mM of a substance buffering in a weak alkaline range, 0.1 to 5 mM of a complexing agent, 0.5 to 5 mM of an agent stabilizing SH groups, at least 100 mM sodium chloride, 45 to 55 % (v/v) glycerol and optionally 0.1 to 5.0 mg/ml bovine serum albumin.

6. Stabilized enzyme solution as claimed in claim 5, wherein it contains 50 mM Hepes/KOH, pH 8.0, 1 mM EDTA, 1 mM dithiothreitol, 300 mM sodium chloride and 50 % (v/v) glycerol.

7. Process for obtaining an enzyme as claimed in one of the claims 1 to 4 from microorganisms of the genus Arthrobacter or Micrococcus, wherein a hydroxyapatite chromatography, anion exchange chromatography and hydrophobic chromatography are carried out after disruption of the cells and separating DNA.

8. Process as claimed in claim 7, wherein DNA is separated by means of Polymin precipitation.

9. Process as claimed in claim 7 or 8, wherein the anion exchange chromatography is carried out on Q-Sepharose ff (high load).

10. Process as claimed in claim 7, 8 or 9, wherein the hydrophobic chromatographic step is carried out on phenyl Sepharose ff.

11. Process as claimed in claim 7, 8, 9 or 10, wherein the microorganism DSM 10239 is used.

12. Use of a thermolabile enzyme with uracil-DNA-glycosylase activity as claimed in one of the claims 1 to 6 to detect DNA containing uracil or to remove the base uracil from DNA, wherein a mixture containing DNA containing uracil is incubated with the enzyme for ca. one minute to 30 minutes at a temperature of 10°C to 30°C and heated for 1 to 10 minutes to 95°C.

13. Use as claimed in claim 12, wherein the DNA containing uracil is a specifically amplified DNA.

14. Use as claimed in claim 12 or 13, wherein when incubated between 30 seconds and four hours at 4°C, uracil-DNA-glycosylase activity can no longer be detected.

15. Kit for amplifying specific nucleic acids or corresponding fragments, wherein the following components are present:
(a) a thermolabile enzyme as claimed in one of the claims 1 to 4 in a suitable storage buffer,
(b) the nucleotide triphosphates dATP, dCTP, dGTP and dUTP,
(c) a thermostable DNA polymerase and
(d) a substance buffering in the pH range of 7.5 to 9.2 (reaction buffer).

16. Kit as claimed in claim 15, wherein the thermolabile enzyme is present at a concentration of 0.1 to 5.0 U/µl.

17. Kit as claimed in claim 15 or 16, wherein the nucleotide triphosphates dATP, dCTP and dGTP are each a third of the concentration of dUTP.

18. Kit as claimed in one of the claims 15 to 17, wherein 2 to 10 U/µl (final concentration) of the thermostable DNA polymerase is present.

19. Kit as claimed in one of the claims 15 to 18, wherein the reaction buffer additionally contains 10 to 100 mM potassium chloride and/or 1.0 to 5.0 mM magnesium chloride.

## Revendications

1. Enzyme thermolabile possédant une activité d'uracile ADN glycosylase, qui présente un poids moléculaire apparent entre 23.000 et 24.000 Dalton, que l'on obtient à partir de micro-organismes du genre Arthrobacter ou Micrococcus avec un degré de pureté d'au moins 95 % (gel SDS) et des temps de demie-vie inférieur à 5 minutes à 40 °C et inférieur à 2 minutes à 45 °C.

2. Enzyme selon la revendication 1, **caractérisée en ce que** la teneur en activités étrangères contaminantes est inférieure à 2 % et **en ce que** l'activité spécifique s'élève à au moins 5 x 10⁴ U/mg de protéine.

3. Enzyme selon la revendication 1 ou 2, **caractérisée en ce que** la température optimale s'élève à 37 °C et le pH optimal s'élève à 6,5.

4. Enzyme selon l'une quelconque des revendications 1, 2 ou 3, **caractérisée en ce que** l'enzyme est isolée de la souche DSM 10239.

5. Solution stabilisée de l'enzyme thermolabile possédant une activité d'uracile ADN glycosylase selon les revendications 1 à 4, **caractérisée en ce que** l'enzyme est contenue dans un mélange constitué par une substance faisant tampon dans le domaine faiblement alcalin à concurrence de 10 à 250 mM, un formateur de complexe à concurrence de 0,1 à 5 mM, un agent stabilisant les groupes SH à concurrence de 0,5 à 5 mM, du chlorure de sodium à concurrence d'au moins 100 mM, du glycérol à concurrence de 45 à 55 % (en volume/volume) et, le cas échéant, de l'albumine de sérum bovin à concurrence de 0,1 à 5,0 mg/ml.

6. Solution enzymatique stabilisée selon la revendication 5, **caractérisée en ce qu'**elle contient Hepes/KOH en une concentration de 50 mM, pH 8,0, EDTA en une concentration de 1 mM, du dithiothréitol en une concentration de 1 mM, du chlorure de sodium en une concentration de 300 mM et du glycérol à concurrence de 50 % (en volume/volume).

7. Procédé pour l'obtention d'une enzyme selon l'une quelconque des revendications 1 à 4, à partir de micro-organismes du genre Arthrobacter ou Micrococcus, **caractérisé en ce que**, après désagrégation des cellules et séparation de l'ADN, on met en oeuvre une chromatographie sur hydroxyapatite, une chromatographie par échange d'anions et une chromatographie hydrophobe.

8. Procédé selon la revendication 7, **caractérisé en ce que** la séparation de l'ADN a lieu par précipitation dans Polymin.

9. Procédé selon la revendication 7 ou 8, **caractérisé en ce que** la chromatographie par échange d'anions a lieu sur de la Q-sépharose FF (high load).

10. Procédé selon la revendication 7, 8 ou 9, **caractérisé en ce qu'**on effectue l'étape de chromatographie hydrophobe sur de la phényl-sépharose FF.

11. Procédé selon la revendication 7, 8, 9 ou 10, **caractérisé en ce qu'**on utilise le micro-organisme DSM 10239.

12. Utilisation d'une enzyme thermolabile possédant une activité d'uracile ADN glycosylase selon l'une quelconque des revendications 1 à 6, pour le décèlement d'ADN uracilique, respectivement pour l'élimination de la base uracile d'ADN, **caractérisée en ce qu'**on incube avec l'enzyme un mélange contenant de l'ADN uracilique pendant un laps de temps de 1 minute à 30 minutes à une température de 10 °C à 30 °C et on chauffe à 95 °C pendant un laps de temps de 1 à 10 minutes.

13. Utilisation selon la revendication 12, **caractérisée en ce que**, en ce qui concerne l'ADN uracilique, il s'agit d'ADN qui a été soumis à une amplification spécifique.

14. Utilisation selon la revendication 12 ou 13, **caractérisée en ce que**, lors de l'incubation, on ne peut plus détecter d'activité d'uracile ADN glycosylase entre 30 secondes et 4 heures à 4 °C.

15. Nécessaire pour la multiplication d'acides nucléiques spécifiques, respectivement de fragments correspondants, **caractérisé en ce qu'**il contient les composants suivants :
(a) une enzyme thermolabile selon l'une quelconque des revendications 1 à 4 dans un tampon de stockage approprié,
(b) les nucléotides triphosphates dATP, dCTP, dGTP et dUTP,
(c) une ADN polymérase thermostable, et
(d) une substance faisant tampon (tampon réactionnel) dans la plage de pH de 7,5 à 9,2.

16. Nécessaire selon la revendication 15, **caractérisé en ce que** l'enzyme thermolabile est présente en une concentration de 0,1 à 5,0 U/µl.

17. Nécessaire selon la revendication 15 ou 16, **caractérisé en ce que** les nucléotides triphosphates dATP, dCTP et dGTP représentent respectivement un tiers de la concentration du dUTP.

18. Nécessaire selon l'une quelconque des revendications 15 à 17, **caractérisé en ce que** l'ADN polymérase thermostable est présente e n une concentration de 2 à 10 U/µl (concentration finale).

19. Nécessaire selon l'une quelconque des revendications 15 à 18, **caractérisé en ce que** le tampon réactionnel contient en outre du chlorure de potassium en une concentration de 10 à 100 mM et/ou du chlorure de magnésium en une concentration de 1,0 à 5,0 mM.
